# DEMANDE DE BREVET EUROPEEN

(11) **EP 1 787 681 A2**
(43) Date de publication de la demande: **23.05.2007**
(21) Numéro de dépôt: 06301070.6
(22) Date de dépôt: 20.10.2006
(51) Int. Cl.: A61Q 1/10, A61K 8/42, A61K 8/34, A61K 8/31, A61K 8/37

(54) **Composition cosmétique texturée par un dérivé bis-urée à phase grasse liquide texturée par un composé bis-urée**

(30) Priorité: 24.10.2005 FR 0553231
(71) Demandeur: L'ORÉAL, 75008 Paris (FR)
(72) Inventeur: Chodorowski-Kimmes, Sandrine, 60300 Senlis (FR); Livoreil, Aude, 75006 Paris (FR); Baghdadli, Nawel, 91300 Massy (FR); Rodriguez, Ivan, 60290 Cauffry (FR)
(74) Mandataire: Le Coupanec, Pascale A.M.P.

(57) **Abrégé**

La présente invention concerne une composition cosmétique comprenant dans un milieu physiologiquement acceptable au moins une phase grasse liquide, texturée par au moins un composé de formule générale (I) : 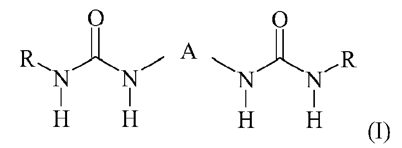 dans laquelle A est un radical phénylène.

## Description

La présente invention concerne le domaine cosmétique et plus particulièrement une composition cosmétique texturée par un dérivé bis-urée à phase grasse liquide texturée par un composé bis-urée.

Pour structurer les huiles et leur donner la texture ou la viscosité souhaitée, l'utilisation d'organogélateurs est bien connue de l'homme de l'art. Les organogélateurs modifient les interactions moléculaires à l'intérieur de l'huile et changent ses caractéristiques physiques et/ou chimiques. Toutefois, la solubilisation de ces molécules organogélatrices dans une huile ou un mélange d'huiles requiert une température souvent élevée, ce qui peut générer des coûts supplémentaires de chauffage et surtout être incompatible avec la présence de molécules thermosensibles. De plus, le gel ainsi obtenu ne présente pas la stabilité nécessaire au cours du temps.

L'utilisation de certaines bis-urées à titre d'organogélateur a été envisagée dans les documents WO 02/47628, JP 2003-064346 et JP 10-236981. Ces documents décrivent entre autre l'utilisation de bis-urées pour texturer des milieux, cosmétiques ou non. Il existe aussi de nombreux articles décrivant des molécules organiques fonctionnalisées par une ou plusieurs urées dont notamment les articles de Bouteiller et al., dans New J. Chem., 2000, 24, 845-848 ; Langmuir, 2002, 18, 7218-7222 et J. Am. Chem. Soc. 2003, 125, 13148-13154 décrivant l'utilisation de certaines bis-urées dans des solvants organiques à l'image du toluène, du tétrachlorure de carbone ou du dodécane à des fins de gélification de ces derniers. Nous pouvons aussi citer comme autre exemple Hanabusa K. et coll. qui décrivent le comportement de molécules bis-urées comme organogélateur (Langmuir 2003, 19(21), 8622-8624). Quant à l'article de Hamilton *et al.* dans Tetrahedron Letters, 1998, 39, 7447-7450, il décrit l'aptitude de certains dérivés bis-urées à se comporter comme des gélifiants dans certains solvants organiques.

Toutefois, toutes les bis-urées décrites dans ces documents ne se solubilisent pas à température ambiante et/ou dans l'ensemble des huiles notamment cosmétiques.

En fait, pour identifier un agent gélifiant capable de texturer de manière satisfaisante une composition cosmétique particulière et apte à être solubilisée à température ambiante dans les huiles de la composition, de nombreux tests doivent généralement être réalisés au préalable.

Il existe donc un besoin de molécules organogélatrices que l'on pourrait qualifier d'universelles dans la mesure où elles seraient efficaces pour texturer, à température ambiante, un grand nombre d'huiles cosmétiques différentes.

La présente invention a précisément pour objectif de proposer de nouvelles bis-urées conformes à ces exigences.

En conséquence, selon l'un de ses aspects, l'invention concerne une composition cosmétique comprenant dans un milieu physiologiquement acceptable au moins une phase grasse liquide contenant au moins un composé lipophile choisi parmi :
- les monoalcools en C₆-C₃₂,
- les alcanes ramifiés en C₆-C₃₂,
- les alcanes linéaires en C₁₃-C₄₈, et
- les huiles bifonctionnelles, comprenant deux fonctions choisies parmi ester et/ou amide, comprenant de 6 à 30 atomes de carbone et 4 hétéroatomes choisis parmi O et N,
caractérisée en ce que ladite phase grasse est texturée par une quantité efficace d'au moins un composé de formule générale (I) : 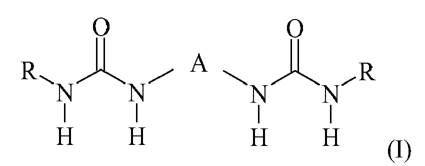 dans laquelle A et R sont tels que définis ci-après.

De manière inattendue, les inventeurs ont découvert que les composés de bis-urée tels que définis ci-dessus étaient des agents gélifiants de choix pour répondre au besoin en molécules organogélatrices universelles exprimé précédemment.

Selon un autre de ses aspects, l'invention concerne l'utilisation d'au moins un composé de bis-urée de formule (I) telle que décrite précédemment pour texturer une composition cosmétique.

Selon encore un autre de ses aspects, l'invention concerne un procédé de maquillage et/ou de soin d'une matière kératinique, notamment de la peau notamment du corps ou du visage, des cils, des sourcils, des ongles et/ou des cheveux, comprenant l'application sur la surface à traiter d'une composition cosmétique comprenant au moins un composé bis-urée de formule (I) telle que décrit précédemment.

### 1) BIS-UREES

Comme précisé ci-dessus, les composés de bis-urée plus particulièrement considérés selon l'invention répondent à la formule générale (I) suivante : 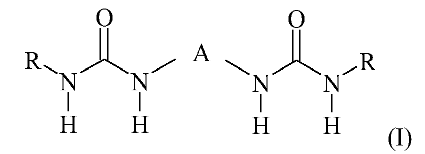 dans laquelle :
- A est un groupement de formule : 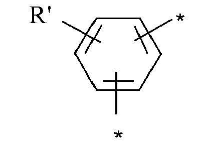 avec R' étant un radical alkyle C₁ à C₄ linéaire ou ramifié et les * symbolisant les points de rattachement du groupement A à chacun des deux atomes d'azote du reste du composé de formule générale (I), et
- R est un radical alkyle en C₆ à C₁₅, mono-ramifié, non cyclique, saturé ou insaturé et dont la chaîne hydrocarbonée est éventuellement interrompue par 1 à 3 hétéroatomes choisis parmi O, S et N, ou
l'un de ses sels ou isomères.

Selon un mode de réalisation préféré de l'invention, le groupement figuré par A est un groupement de formule : 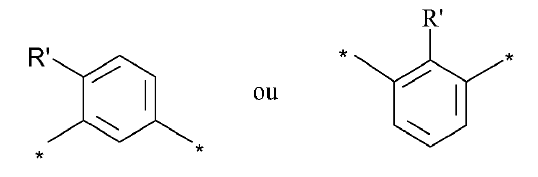 avec R' et les * étant tels que définis précédemment.

En particulier, R' peut être un groupement méthyle, et le groupement A est alors plus particulièrement un groupement de formule : 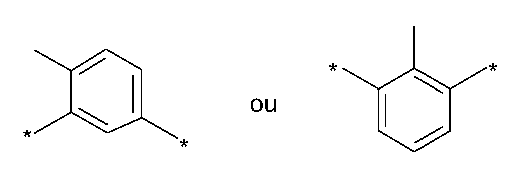 avec les * étant tel que défini précédemment.

Selon un premier mode de réalisation de l'invention, R peut être choisi parmi les radicaux mono-ramifiés de formule générale CₙH₂ₙ₊₁, n étant un entier variant de 6 à 15, en particulier de 7 à 9 voire égal à 8.

Ainsi, les deux groupements R du composé de formule (I) peuvent figurer respectivement un groupement : 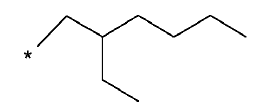 avec * symbolisant le point de rattachement de chacun des groupements R à chacun des atomes d'azote du reste du composé de formule générale (I).

Selon un deuxième mode de réalisation de l'invention, R peut être choisi parmi les radicaux mono-ramifiés de formule générale Cₘ₋ₚH₂ₘ₊₁₋₂ₚXₚ, p étant égal à 1, 2 ou 3, de préférence égal à 1, m étant un entier variant de 6 à 15, de préférence de 10 à 14, en particulier de 10 à 12, voire égal à 11 et X représentant les atomes de soufre et/ou d'oxygène, en particulier les atomes d'oxygène.

Plus particulièrement, R peut être un radical de formule C_{m'}H_{2m'}X-(C_{p'}H_{2p'}X')ᵣ-CₓH₂ₓ₊₁, dans laquelle X et X' sont indépendamment l'un de l'autre un atome d'oxygène ou de soufre, de préférence d'oxygène, r vaut 0 ou 1, m', p' et x sont des entiers tels que leur somme varie de 6 à 15, en particulier de 10 à 12, voire est égale à 11 et étant entendu qu'au moins une des chaînes carbonées C_{m'}H_{2m'}, C_{p'}H_{2p'}, ou CₓH₂ₓ₊₁ est ramifiée.

De préférence c'est la chaîne CₓH₂ₓ₊₁ qui est ramifiée, de préférence r égal 0, de préférence m' est un entier variant de 1 à 10, notamment de 2 à 6, en particulier égal à 3, et/ou de préférence x est un entier variant de 4 à 16, notamment de 6 à 12, en particulier égal à 8.

Ainsi, les deux groupements R du composé de formule (I) peuvent figurer respectivement un groupement : 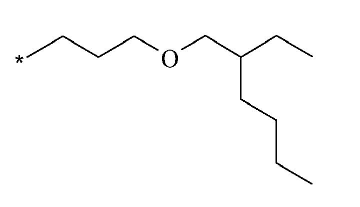 avec * symbolisant le point de rattachement de chacun des groupements R à chacun des atomes d'azote du reste du composé de formule générale (I).

De tels composés peuvent être présents dans les compositions selon l'invention en mélanges avec des isomères, notamment des isomères de position sur le groupement A, notamment dans des proportions 95/5 ou 80/20.

Comme il ressort des exemples ci-après, la présence de l'un ou l'autre de ses radicaux dans la molécule de formule générale (I) s'avère particulièrement avantageuse pour conférer un caractère universel, au sens de l'invention, aux dérivés bis-urée correspondants.

A titre représentatif et non limitatif des composés convenant tout particulièrement à l'invention, on peut plus particulièrement citer les composés suivants, utilisés purs ou en mélange : 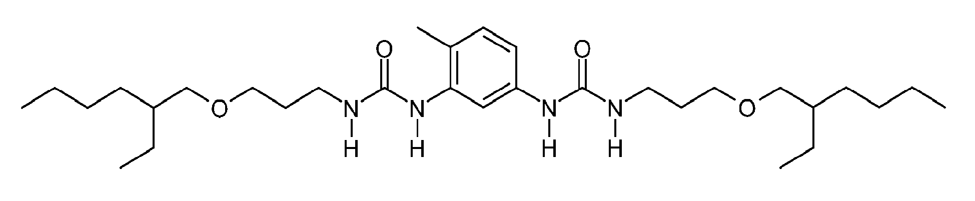 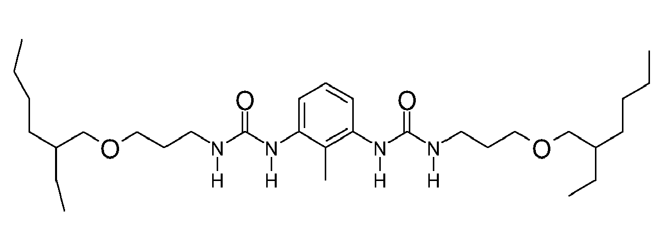 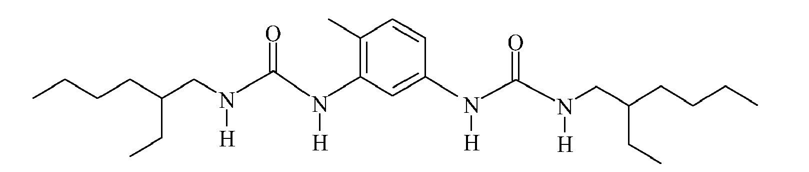 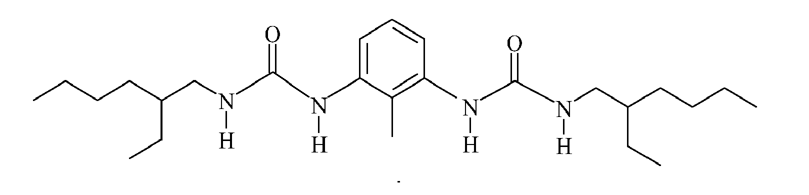 et leurs sels.

Par « conviennent particulièrement dans le cadre de l'invention », on entend que le composé de formule générale (I), seul ou en mélange en toutes proportions, peut se solubiliser dans une grande diversité d'huiles et qu'il s'avère efficace pour texturer ladite huile ou mélange d'huiles considéré(e) et donc lui conférer les propriétés physiques et/ou chimiques souhaitées.

Par « physiologiquement acceptable », on désigne un milieu dénué de toxicité et compatible avec une application sur la peau, les lèvres et/ou les phanères d'êtres vivants et en particulier d'êtres humains. En conséquence, les compositions selon l'invention sont dénuées de composés incompatibles et/ou non tolérés pour une application sur la peau, les lèvres et/ou les phanères.

Au sens de la présente invention, le terme « quantité efficace » désigne la quantité nécessaire et suffisante pour obtenir la texturation de l'huile ou mélange d'huiles considéré dans la composition selon l'invention.

Par « phase grasse liquide texturée », on entend que la phase grasse prend l'état d'un gel ou d'un liquide épaissi. Elle peut s'écouler sous son propre poids. Elle peut se déformer à volume constant si on exerce une contrainte.

Cette texturation se traduit en particulier par une augmentation de la viscosité due notamment à l'introduction d'au moins un composé de formule générale (I).

Par exemple, les compositions selon l'invention peuvent contenir de 0,01 à 20 % en poids, notamment de 0,1 à 15 %, voir de 1 à 10, encore mieux de 2 à 8 %, en poids de composé(s) de formule (I) par rapport au poids total de la composition.

La quantité efficace de composé(s) de composé(s) de formule (I), peut représenter de 0,01 à 20 %, notamment de 0,05 à 10 %, par exemple de 0,1 à 5 %, voire de 0,05 à 3 % en poids de la phase grasse liquide.

Pour des raisons évidentes, cette quantité efficace est susceptible de varier significativement selon d'une part la nature du substituant R du dérivé bis-urée, sa position, le fait qu'il soit utilisé à l'état pur ou en mélange avec d'autres dérivés bis-urée de formule (I) ou non, et d'autre part la nature de la phase grasse liquide.

D'une manière générale, le composé de formule générale (I) selon l'invention dérive de la réaction entre au moins un di-isocyanate de formule : 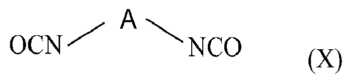 et une amine primaire de formule : 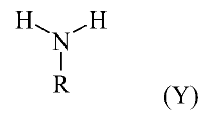 avec A et R tels que définis précédemment.

Le cas échéant, les différents diisocyanates (X) peuvent être des isomères de positions du susbtituant R' sur le groupement A, notamment dans des proportions 95/5 ou 80/20.

De préférence l'amine (Y) utilisée est dans un rapport molaire de 2 à 3 équivalents, notamment 2,1 à 2,5, voire 2,2 équivalents pour un équivalent de diisocyanate(s) (X). Le schéma réactionnel général est le suivant :

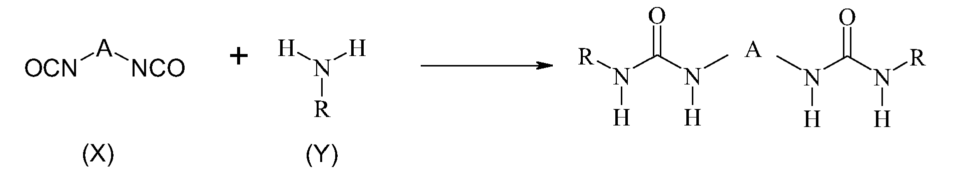

La réaction est généralement effectuée sous atmosphère inerte par exemple sous argon, en milieu anhydre avec par exemple une température du milieu réactionnel qui est maintenue entre 15 °C et 40 °C, de préférence entre 18 °C et 25 °C.

Le(s) diisocyanate(s) (X) peut(peuvent) être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30 % en poids, de préférence de 2 à 20 %, voire de 4 à 10 % en poids.

Une solution contenant l'amine (Y) est généralement préparée dans le même solvant que le(s) diisocyanate(s) (X) à une concentration allant par exemple de 0,1 à 99,9 % en masse. La température du milieu réactionnel ne doit préférentiellement pas dépasser 40 °C et la concentration de l'amine ainsi que la vitesse d'addition de la solution contenant l'amine (Y) doivent donc être préférentiellement ajustées à cette nécessité. Le milieu réactionnel peut être laissé sous agitation par exemple pendant 30 min à 12 heures. Le contrôle de l'avancement de la réaction peut être réalisé par spectrométrie infra-rouge (notamment en observant la disparition de la bande NCO entre 2250 et 2280 cm⁻¹). Par exemple, en fin de réaction, on verse le milieu réactionnel dans une grande quantité d'eau acide (notamment un pH 3-4 par HCl). On obtient alors un précipité qui est filtré, lavé par exemple plusieurs fois notamment à l'eau et séché sous pression réduite, notamment sous vide ou lyophilisé. Le précipité correspond aux composés de formule (I) attendu(s), et peut être caractérisé par spectrométrie RMN (¹H et ou ¹³C) et/ou par HPLC et peut être utilisé tel quel pour la texturation du milieu huileux considéré.

La bis-urée ou le mélange de bis-urées est avantageusement soluble à une température inférieure ou égale à 50 °C, voire inférieure ou égale à 30 °C, et notamment à température ambiante, dans la phase grasse liquide à texturer.

### 2) PHASE GRASSE LIQUIDE

Par phase grasse liquide, on entend au sens de l'invention une phase grasse liquide à température ambiante (25 °C) et pression atmosphérique (760 mm de Hg), composée d'un ou plusieurs composé lipophiles tels que définis ci-dessus, et éventuellement d'un ou plusieurs corps gras liquides à température ambiante, appelés aussi huiles, compatibles entre eux et physiologiquement acceptables.

La phase grasse liquide peut représenter de 1 à 99,99 % en poids de la composition, voire de 5 à 95 %, notamment de 10 à 90 %, en particulier de 20 à 50 % en poids de la composition.

Comme énoncé précédemment, les compositions cosmétiques comprennent une phase grasse comprenant au moins un composé choisi parmi :
- les monoalcools en C₆-C₃₂,
- les alcanes ramifiés en C₆-C₃₂,
- les alcanes linéaires en C₁₃-C₄₈, et
- les huiles bifonctionnelles, comprenant deux fonctions choisies parmi ester et/ou amide et comprenant de 6 à 30 atomes de carbone et 4 hétéroatomes choisis parmi O et N.

Au sens de l'invention, de tels composés seront encore appelés « composés lipophiles ».

Selon un mode de réalisation particulier de l'invention, le monoalcool en C₆-C₃₂ est un monoalcool en C₈-C₂₈, mieux en C₁₂-C₂₆ et est préférentiellement l'octyldodécanol.

Selon un autre mode de réalisation particulier, l'alcane ramifié en C₆-C₃₂ est un alcane ramifié en C₈-C₂₈, mieux en C₁₂-C₂₆ et est préférentiellement l'isododécane ou le parléam de formule -(CH₂-CH(CH₃)ₙ- avec n étant un entier variant par exemple de 4 à 8.

Selon un aspect particulier de l'invention, l'alcane linéaire en C₁₃-C₄₈ est un alcane linéaire en C₁₈-C₄₀, mieux en C₂₀-C₃₂.

Selon encore un autre mode de réalisation de l'invention, l'huile bifonctionnelle comprend de 8 à 28 atomes de carbone, mieux de 10 à 24 carbones, et/ou les fonctions amide et ester de l'huile sont dans la chaîne, et en particulier l'huile bifonctionnelle peut être le N-lauroylsarcosinate d'isopropyle de formule suivante : 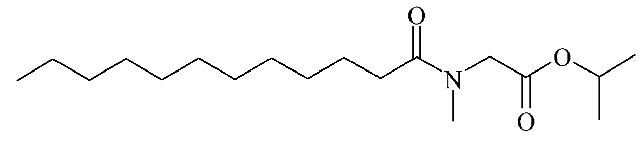 et plus particulièrement celui commercialisé sous la dénomination Eldrew SL-205^{®} d'Ajinomoto.

Le ou les composé(s) lipophile(s) représente(nt) notamment de 20 à 100 %, voire de 40 à 99 %, notamment de 60 à 95 % en poids de la phase grasse liquide.

En plus du ou des composé(s) lipophile(s) contenu(s) dans la phase grasse liquide, cette dernière peut en outre contenir une ou mélange d'huiles annexes choisies parmi les huiles ester, des huiles non siliconées et/ou des huiles siliconées.

Cette/Ces huile(s) annexe(s) peut (peuvent) représenter de 0,1 à 80 %, par exemple de 1 à 60 %, voire de 5 à 40 % en poids de la phase grasse liquide.

### a. Huile ester additionnelle

Selon une variante de l'invention, la phase grasse liquide peut comprendre en outre une huile, dite « huile ester », qui est notamment choisie parmi les esters des acides monocarboxyliques avec les monoalcools et polyalcools.

Avantageusement, ledit ester répond à la formule (II) suivante :

R₁-CO-O-R₂ (II)

où
- R₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
- R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.

Par « éventuellement substitué », on entend que R₁ et/ou R₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.

Des exemples des groupes R₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

Des exemples d'esters utilisables dans les phases grasses des compositions de l'invention sont plus particulièrement des esters en C₈-C₄₈, éventuellement incorporant dans leur chaîne hydrocarbonée un ou plusieurs hétéroatomes parmi N et O et/ou une ou plusieurs fonctions carbonyle.

A titre illustratif et non limitatif de ces esters, on peut plus particulièrement citer l'huile de purcellin (octanoate de cétostéaryle), l'isononanoate d'isononyle, le myristate d'isopropyle, le palmitate d'éthyl-2-hexyle, le stéarate d'octyl-2-dodécyle, l'érucate d'octyl-2-dodécyle, le l'isostéarate d'isostéaryle, et les heptanoates, octanoates, décanoates ou ricinoléates d'alcools ou de polyalcools, par exemple d'alcools gras.

De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.

### b. Huile non siliconée additionnelle

La phase grasse liquide des compositions selon l'invention peut contenir en outre une ou plusieurs huiles non siliconées distinctes des composés lipophiles et des huiles esters décrits précédemment. Ces huiles non siliconées peuvent être choisies dans le groupe des huiles hydrocarbonées et des éthers volatils.

L'huile non siliconée peut aussi être choisie parmi les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse en C₆ à C₄₀,
- les acides gras en C₈-C₃₂, comme l'acide oléique, linoléique ou linolénique, et
- leurs mélanges.

La phase grasse liquide peut encore contenir des huiles apolaires autres que les composés lipophiles précédemment cités telles que les hydrocarbures ou fluorocarbures, linéaires ou ramifiés, d'origine synthétique ou minérale, comme les huiles de paraffine (telles que les isoparaffines en C₈-C₁₆ ou l'isohéxadécane) et leurs dérivés, la vaseline, les polydécènes, les polyisobutènes hydrogénés tel que le squalane, et leurs mélanges.

### c. Huile siliconée

Selon un mode de réalisation particulier de l'invention, la phase grasse liquide peut également comprendre une ou plusieurs huile(s) siliconée(s).

Ces huiles peuvent être volatiles ou non volatiles. Au sens de l'invention, les huiles volatiles présentent à température ambiante (25 °C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa).

L'huile siliconée peut être choisie parmi les huiles siliconées volatiles linéaires ou cycliques, telles que les polydiméthyl siloxanes (PDMS) linéaires ou cycliques ayant de 3 à 7 atomes de silicium.

A titre d'exemple de telles huiles volatiles, on peut citer les composés tels que l'octyltriméthicone, l'hexyltriméthicone, la décaméthylcyclopentasiloxane (ou D5), l'octaméthylcyclotétrasiloxane, la cyclotétradiméthylsiloxane (ou D4), la dodécaméthylcyclohexasiloxane (ou D6), la décaméthyltétrasiloxane (ou L4) KF 96 A de Shin Etsu, la PDMS (polydiméthylsiloxane) DC 200 (1,5 cSt) de Dow Corning, la PDMS DC 200 (2 cSt) de Dow Corning, la PDMS DC 200 (5 cSt) de Dow Corning, la PDMS DC 200 (3 cSt) de Dow Corning, et/ou leurs mélanges.

On peut également citer l'heptaméthyloctyltrisiloxane, le dodécaméthylpentasiloxane, le polyméthylcétyldiméthylsiloxane et/ou leurs mélanges.

L'huile siliconée volatile peut aussi être choisie dans le groupe des huiles siliconées fluorées telles que les silicones à groupes alkyle et perfluoralkyle, les silicones à groupes latéraux oxyéthylénés/oxypropylénés (OE/PP) et à groupes perfluorés, les silicones à groupes latéraux perfluorés ou polyfluorés et à groupes latéraux glycérolés, et les perfluoroalkylméthylphénylsiloxanes.

Les huiles siliconées non volatiles peuvent être des polydiméthylsiloxanes, des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.

Les huiles siliconées non volatiles utilisables peuvent être en particulier les polydiméthylsiloxanes (PDMS) non volatils, linéaires, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et/ou leurs mélanges.

Les inventeurs ont constaté que les associations d'au moins une bis-urée de formule (I) dans laquelle les deux groupements R figurent respectivement par un groupement : 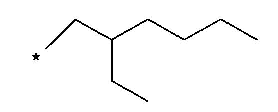 avec * symbolisant le point de rattachement de chacun des groupements R à chacun des atomes d'azote du reste du composé de formule générale (I),
avec au moins un composé lipophile tel que défini ci-dessus, et notamment une phase grasse contenant de l'isododécane, de l'octyldodécanol, du N-lauroyl sarcosinate d'isopropyle, du parléam et/ou un de leurs mélanges, donnent des résultats particulièrement satisfaisants au sens de l'invention.

De même que les associations d'au moins une bis-urée de formule (I) dans laquelle les deux groupements R figurent respectivement par un groupement : 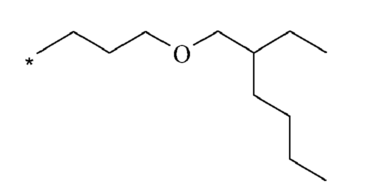 avec * symbolisant le point de rattachement de chacun des groupements R à chacun des atomes d'azote du reste du composé de formule générale (I),
avec au moins un composé lipophile tel que défini ci-dessus, et notamment une phase grasse contenant de l'isododécane, de l'octyldodécanol, du parléam, du N-lauroyl sarcosinate d'isopropyle et/ou un de leurs mélanges donnent également des résultats particulièrement satisfaisants au sens de l'invention.

### 3) AUTRES CORPS GRAS

Les compositions selon l'invention peuvent en outre comprendre au moins un corps gras solide, qui peut être choisi parmi les cires et/ou les composés pâteux.

Plus particulièrement, les compositions selon l'invention peuvent comprendre de 0,1 % à 40 % en poids, notamment de 0,1 % à 30 % en poids, et plus particulièrement de 0,5 % à 25 % en poids de corps gras solide(s) par rapport au poids total de la composition.

Par cire au sens de la présente invention, on entend un composé lipophile, solide à température ambiante (25 °C), à changement d'état solide/liquide réversible, ayant un point de fusion supérieur ou égal à 30 °C, mieux supérieur à 45 °C, et pouvant aller jusqu'à 120 °C.

Comme cire utilisable dans la première composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les compositions peuvent également contenir une cire micronisée, appelée également micro cire.

A titre indicatif, les compositions selon l'invention peuvent contenir de 0,1 à 50 % en poids et mieux de 1 à 30 % en poids de cire par rapport à leur poids total.

### 4) PHASE AQUEUSE

La composition peut le cas échéant comprendre au moins une phase aqueuse qui peut ou non être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄ et les aldéhydes en C₂-C₄.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids, par rapport au poids total de la composition.

Selon un autre mode de réalisation conforme à l'invention, la réalisation est exempte d'eau.

### 5) MATIÈRES COLORANTES

Selon un mode de réalisation, la composition selon l'invention peut contenir en outre au moins une matière colorante, organique ou inorganique, notamment de type pigments ou nacres.

Selon un autre mode de réalisation, la composition selon l'invention peut contenir en outre au moins une matière colorante choisie parmi les colorants lipophiles, les colorants hydrophiles, les pigments, les nacres, les matériaux à effet optique spécifique, et leurs mélanges.

Par « nacres », il faut comprendre des particules colorées de toute forme, irisées ou non, notamment produites par certains mollusques dans leur coquille ou bien synthétisées et qui présentent un effet de couleur par interférence optique.

Cette matière colorante peut être présente à raison de 0,01 à 50 % en poids par rapport au poids total de la composition, en particulier de 0,5 à 40 % et plus particulièrement de 5 à 25 %, et notamment de 0,01 à 20 %, et en particulier de 0,1 à 10 %, voire de 2 à 5 % en poids par rapport au poids total de la composition.

### 6) AUTRES ADDITIFS

La composition de l'invention peut comprendre, en outre, tout ingrédient usuellement utilisé dans le domaine concerné.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

On peut notamment citer les charges, les vitamines, les épaississants distincts de ceux répondant à la formule générale (I), les gélifiants différents de ceux répondant à la formule (I), les oligo-éléments, les adoucissants, les hydratants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les actifs cosmétiques en général (anti-âge, canitie, actifs anti-chute, actifs de soin du visage et/ou du corps, les agents amincissants, etc...), les oxydants et les réducteurs (pour la coloration) des actifs dermatologiques et/ou leurs mélanges.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de stick ou bâton.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières ; un produit pour les lèvres tel qu'un rouge à lèvres ou un soin des lèvres; un produit anti-cernes ; un blush, un mascara, un eye-liner ; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

La composition selon l'invention peut être une composition de protection ou de soin de la peau du visage, du cou, des mains ou du corps, notamment une composition anti-rides, anti-fatigue, anti-âge, permettant de donner un coup d'éclat à la peau, une composition hydratante ou traitante; une composition anti-solaire ou après-soleil ou de bronzage artificiel. La composition selon l'invention peut être également un produit capillaire, notamment pour la coloration, le soin, l'hygiène des cheveux, le maintien de la coiffure ou la mise en forme des cheveux. Les compositions capillaires sont de préférence des shampooings, des gels, des lotions de mise en plis, des lotions pour le brushing, des compositions de soin ou d'hygiène capillaire ou des compositions de fixation et de coiffage telles que les laques ou spray. Les produits capillaires peuvent être conditionnés sous diverses formes (gels, pâtes, crèmes...). Il peut également s'agir de lotions notamment dans des vaporisateurs, des flacons-pompe ou dans des récipients aérosol afin d'assurer une application de la composition sous forme vaporisée ou sous forme de mousse.

La composition selon l'invention peut être fabriquée par les procédés connus, généralement utilisés dans le domaine cosmétique ou dermatologique.

L'invention a aussi pour objet un procédé de traitement cosmétique, notamment de maquillage, de nettoyage, de protection solaire, de mise en forme, de coloration, ou de soin, des matières kératiniques, notamment de la peau du corps ou du visage, des ongles, des sourcils, des cheveux et/ou des cils, comprenant l'application sur lesdites matières d'une composition cosmétique telle que définie précédemment.

Les exemples figurant ci-après sont produits à titre illustratif et non limitatif du domaine de l'invention.

### MATERIEL ET METHODE

Les exemples 1 à 3 figurant ci-après diffèrent par la nature chimique de l'amine (Y) utilisée dans le mélange réactionnel ainsi que par le diisocyanate (X) de départ. Les symboles (X) et (Y) figurent les réactifs du schéma réactionnel général présenté précédemment.

Sans indication contraire, les composés de formule générale (I) considérés dans chacun des exemples 1 à 3 ont été préparés selon un même mode opératoire tel que décrit ci-après. Les quantités spécifiques retenues pour les composés X et Y sont précisées dans les exemples ci-après.

On réalise le mélange de toluilène diisocyanate en solution dans le THF avec 2,2 équivalents d'amine est préparé par réaction sous argon. La réaction est effectuée sous atmosphère inerte en milieu anhydre avec une température du milieu réactionnel qui est maintenue entre 15 °C et 40 °C.

Parallèlement, on prépare une solution d'amine (Y) dans de la THF. La température du milieu réactionnel ne devant pas dépasser 40 °C, la concentration de l'amine et la vitesse d'addition de la solution d'amine (Y) sont ajustés à cette nécessité. On laisse le milieu réactionnel sous agitation en contrôlant l'avancement de la réaction par spectrométrie infra-rouge (disparition de la bande NCO entre 2250 et 2280 cm⁻¹).

Une fois que le diisocyanate a complètement réagi, le mélange réactionnel est additionné à de l'eau acidifiée à un pH 3 avec de l'acide chlorhydrique, le précipité obtenu est filtré, lavé plusieurs fois à l'eau et enfin séché. Une poudre blanche est obtenue et utilisée telle quelle après analyse (HPLC couplée à la masse).

### EXEMPLE 1 :

(X) = 50 g de toluilène diisocyanate en mélange d'isomère 2,4 et d'isomère 2,6 en proportion 95/5,
(Y) = 79,6 g de (2-éthyl-héxylamine) :

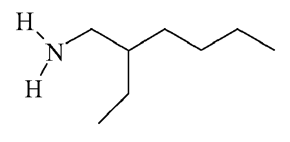

On obtient le composé (I) sous forme d'un mélange de composés de formules suivantes :

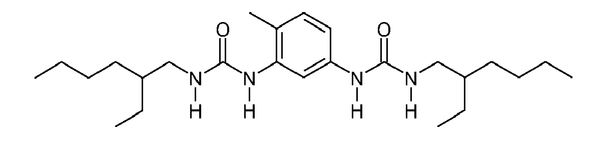

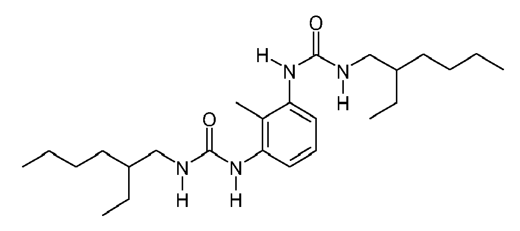

Le rapport molaire des isomères est déterminé par spectrométrie RMN ¹H et/ou par HPLC. Les spectres RMN sont conforme aux structures attendues. Le mélange de produit obtenu se présente sous forme d'une poudre blanche.

### EXEMPLE 2 :

(X) = 19,8 g detoluilène diisocyanate en mélange d'isomère 2,4 et d'isomère 2,6 en proportion 95/5.
(Y) = 47,2 g de 3-(2-éthylhexyloxy)propylamine :

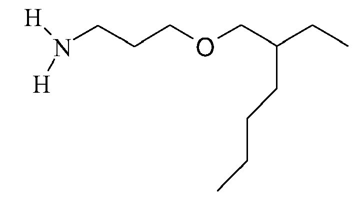

On obtient le composé (I) sous forme de mélange de formules suivantes :

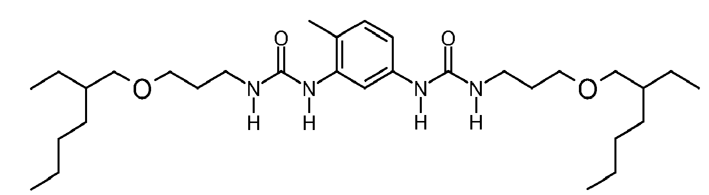

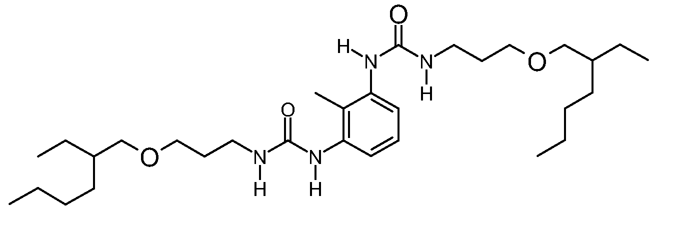

Le rapport molaire des isomères est déterminé par spectrométrie RMN ¹H et/ou par HPLC. Les spectres RMN sont conformes aux structures attendues. Le mélange de produit obtenu se présente sous forme d'une poudre blanche.

### EXEMPLE 3 :

(X) = 8,7 g de toluilène diisocyanate en mélange d'isomère 2,4 et d'isomère 2,6 en proportion 80/20.
(Y) = 14,2 g de 2-éthyl-héxylamine :

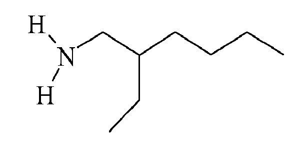

On obtient le composé (I) sous forme de mélange de formules suivantes :

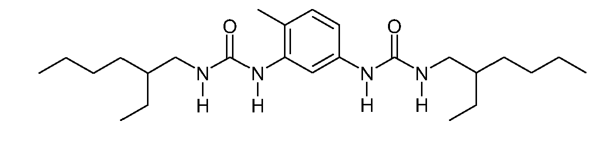

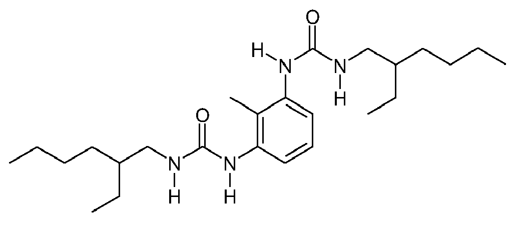

Le rapport molaire des isomères est déterminé par spectrométrie RMN ¹H et/ou par HPLC. Les spectres RMN sont conformes aux structures attendues. Le mélange de produits obtenu se présente sous forme d'une poudre blanche.

### EXEMPLE 4 :

A titre comparatif, il a été préparé les mélanges de bis-urées suivants :

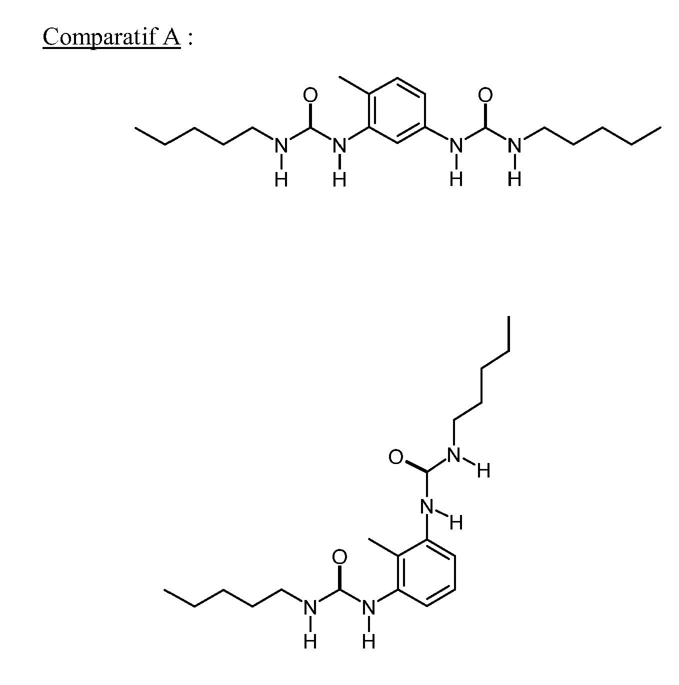

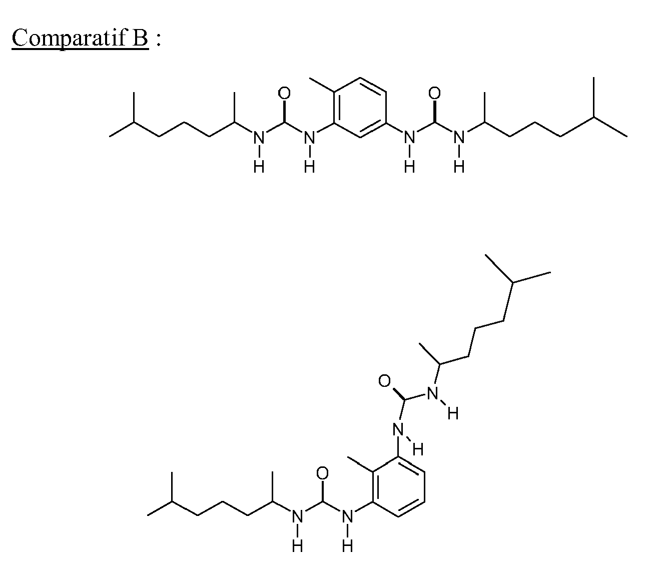

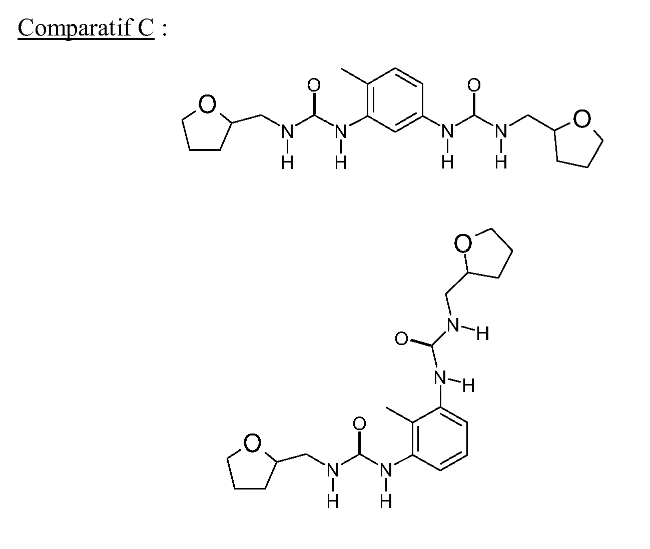

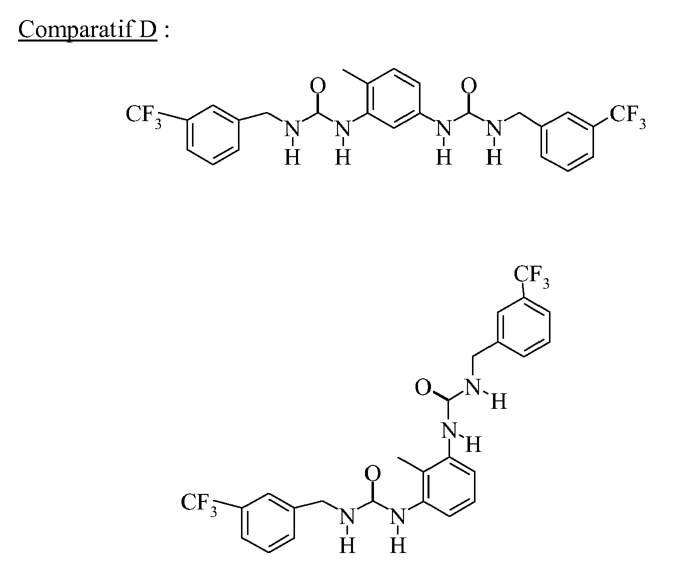

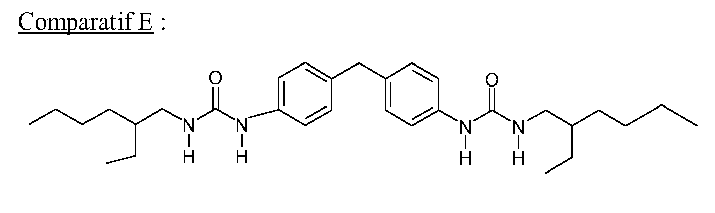

### EXEMPLE 5 :

Les produits ou mélanges de produits obtenus à chacun des exemples 1, 2 et 3 et des composés comparatifs A, B, C, D et E sont testés à raison de 1 à 2 % en poids dans 100 ml des composés lipophiles considérés pour leurs propriétés texturantes à température ambiante vis-à-vis d'une liste de composés lipophiles (isododécane, octyldodécanol, parléam et N-lauroyl sarcosinate d'isopropyle).

Les propriétés texturantes sont jugées satisfaisantes si le composé ou mélange de composés se solubilise à température ambiante dans ledit composé lipophile et, le cas échéant, s'il augmente la viscosité de ce dernier.

On note que seuls les mélanges des exemples 1, 2 et 3 comprenant un composé conforme à l'invention s'avèrent solubles à température ambiante à des concentrations jusqu'à 10 % en poids dans l'isododécane, l'octyldodécanol, le parléam, et augmentent la viscosité de ces derniers.

En revanche, les dérivés des exemples comparatifs A, B, C, D et E ne s'avèrent pas solubles à 1 % en poids dans ces composés lipophiles, même à 80 °C.

De la même manière, on observe que le mélange de l'exemple 1 ou 3 est soluble à température ambiante à des concentrations allant de 0.5 à 10 % dans le N-lauroyl sarcosinate d'isopropyle et augmente la viscosité de ce dernier.

Par contre, les dérivés des exemples comparatifs A et B ne sont pas solubles à 1 % en poids dans ce composé lipophile même à 80 °C et ne peuvent donc pas servir de structurant dans ce composé.

### EXEMPLE 6 :

A titre d'exemple de mélange d'huile, le composé (I) obtenu dans l'exemple 1 est solubilisé à température ambiante à 2 % dans l'isododécane (0.5 g du mélange dans 25 g d'isododécane). Une fois un gel homogène, 25 g d'huile siliconé D5 est ajouté. On obtient au final un gel stable dans le temps dans un mélange isododécane/D5.

### EXEMPLE 7 :

A titre d'exemple de mélange d'huile, le composé (I) obtenu dans l'exemple 3 est solubilisé à température ambiante à 2 % dans l'isododécane (0.5 g du mélange dans 25 g d'isododécane). Lorsqu'un gel homogène est obtenu, 25 g d'huile siliconé D5 y sont ajoutés. On obtient au final un gel stable dans le temps dans un mélange isododécane/D5.

### EXEMPLE 8 : mascara

La composition suivante est réalisée :

| Nature des constituants | Concentration % en poids |
|---|---|
| Cire d'Abeille | 9.9 |
| Cire de Carnauba | 4.52 |
| Paraffine | 2.18 |
| Mexomère PQ¹ | 0 |
| Mexomère PP² | 0.75 |
| Composé de l'Exemple 1 | 5 |
| Oxyde de fer noir | 2.5 |
| Bleu Outre mer | 2.1 |
| Conservateur | 0.2 |
| isododécane | qsp |

| | |
|---|---|
| ¹ Stéarate d'allyl/ VA COPOLYMER ² Polyvinyl laurate | |

Un gel aux qualités cosmétiques tout à fait satisfaisantes est obtenu. On obtient un mascara possédant les qualités cosmétiques requises.

## Revendications

1. Composition cosmétique comprenant dans un milieu physiologiquement acceptable au moins une phase grasse liquide, contenant au moins un composé lipophile choisi parmi :
- les monoalcools en C₆-C₃₂,
- les alcanes ramifiés en C₆-C₃₂,
- les alcanes linéaires en C₁₃-C₄₈, et
- les huiles bifonctionnelles, comprenant deux fonctions choisies parmi ester et/ou amide, comprenant 6 à 30 atomes de carbone et 4 hétéroatomes choisis parmi O et N,
**caractérisée en ce que** ladite phase grasse est texturée par une quantité efficace d'au moins un composé de formule générale (I) : 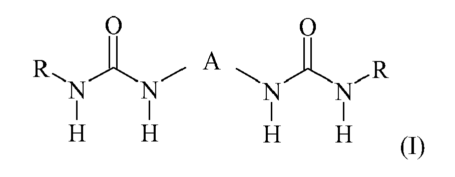 dans laquelle :
- A est un groupement de formule : 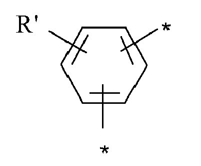 avec R' étant un radical alkyle C₁ à C₄ linéaire ou ramifié et les * symbolisant les points de rattachement du groupement A à chacun des deux atomes d'azote du reste du composé de formule générale (I), et
- R est un radical alkyle en C₆ à C₁₅, mono-ramifié, non cyclique, saturé ou insaturé et dont la chaîne hydrocarbonée est éventuellement interrompue par 1 à 3 hétéroatomes choisis parmi O, S et N, ou
l'un de ses sels ou isomères.

2. Composition selon la revendication 1, **caractérisée en ce que** le groupement figuré par A est un groupement de formule : 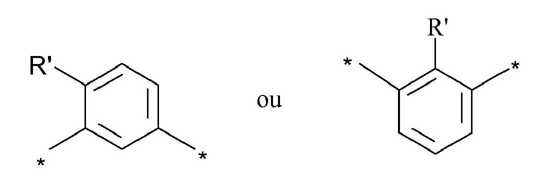 avec R' et * étant tels que définis en revendication 1.

3. Composition selon la revendication précédente, **caractérisée en ce que** le groupement A est un groupement de formule : 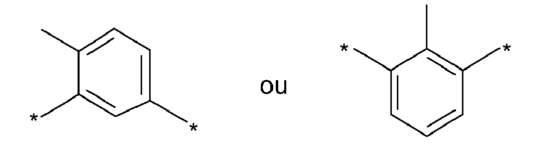 avec * étant tel que défini en revendication 1.

4. Composition selon l'une quelconque des revendications précédentes, **caractérisée en ce que** le groupement R est de formule générale CₙH₂ₙ₊₁ avec n étant un entier variant de 6 à 15.

5. Composition selon la revendication précédente, **caractérisée en ce que** n varie de 7 à 9 et en particulier n égal 8.

6. Composition selon la revendication précédente, **caractérisée en ce que** les deux groupements R figurent respectivement un groupement : 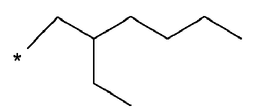 avec * symbolisant le point de rattachement de chacun des groupements R à chacun des atomes d'azote du reste du composé de formule générale (I).

7. Composition selon l'une quelconque des revendications 1 à 3, **caractérisée en ce que** le groupement R est de formule générale Cₘ₋ₚH₂ₘ₊₁₋₂ₚXₚ, p étant égal à 1, 2 ou 3, m étant un entier variant de 6 à 15 et X représentant les atomes de soufre et/ou d'oxygène, en particulier les atomes d'oxygène.

8. Composition selon la revendication précédente, **caractérisée en ce que** le groupement R est de formule C_{m'}H_{2m'}X-(C_{p'}H_{2p'}X')ᵣ-CₓH₂ₓ₊₁, dans laquelle X et X' sont indépendamment l'un de l'autre un atome d'oxygène ou de soufre de préférence d'oxygène, r vaut 0 ou 1 et m', p' et x sont des entiers tels que leur somme varie de 6 à 15.

9. Composition selon la revendication précédente, **caractérisée en ce que** r égal 0.

10. Composition selon l'une quelconque des deux revendications précédentes, **caractérisé en ce que** c'est la chaîne CₓH₂ₓ₊₁ qui est ramifiée.

11. Composition selon l'une quelconque des revendications 8 à 10, **caractérisée en ce que** m' est un entier variant de 1 à 10, notamment de 2 à 6, en particulier égal à 3.

12. Composition selon l'une quelconque des revendications 8 à 11, **caractérisée en ce que** x est un entier variant de 4 à 16, notamment de 6 à 12, en particulier égal à 8.

13. Composition selon l'une quelconque des revendications 7 à 12, **caractérisée en ce que** m ou la somme de m', p' et x varie de 10 à 12 en particulier est égal à 11.

14. Composition selon la revendication précédente, **caractérisée en ce que** les deux groupements R figurent respectivement un groupement : 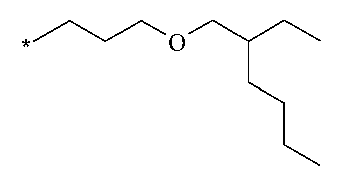 avec * tel que défini en revendication 6.

15. Composition selon l'une quelconque des revendications précédentes, **caractérisée en ce qu'**elle comprend au moins un composé choisi parmi : 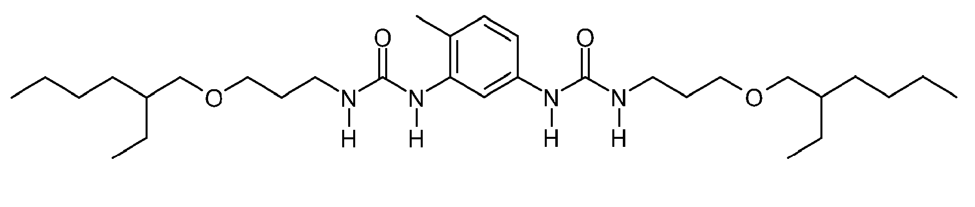 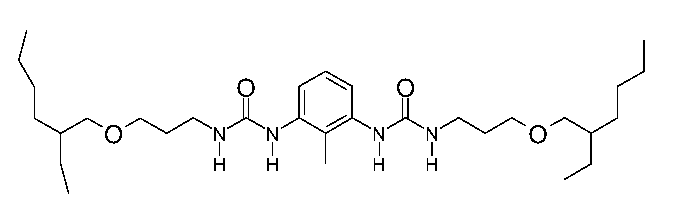 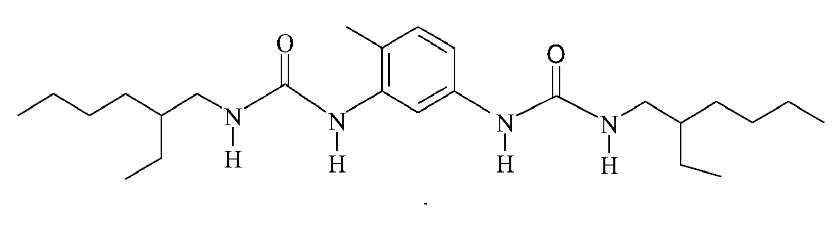 et 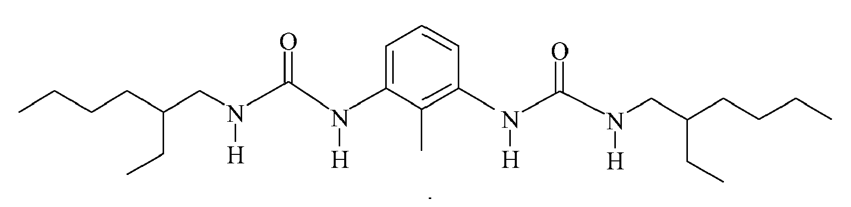 leurs sels et leurs isomères.

16. Composition selon l'une quelconque des revendications précédentes, **caractérisée en ce qu'**elle comprend 2 à 8 % en poids en composé(s) de formule générale (I) par rapport au poids total de la composition.

17. Composition selon l'une quelconque des revendications précédentes, **caractérisée en ce que** le(s) composé(s) de formule générale (I) représente(nt) moins de 3 % en poids de la phase grasse liquide.

18. Composition selon l'une quelconque des revendications précédentes, **caractérisée en ce que** le monoalcool en C₆-C₃₂ est un monoalcool en C₈-C₂₈ voire en C₁₂-C₂₆ et est préférentiellement l'octyldodécanol. 1.

19. Composition selon l'une quelconque des revendications précédentes, **caractérisée en ce que** l'alcane ramifié en C₆-C₃₂ est un alcane ramifié en C₈-C₂₈, mieux en C₁₂-C₂₆ et préférentiellement l'isododécane ou le parléam de formule -(CH₂-CH(CH₃)ₙ- avec n étant un entier variant de 4 à 8.

20. Composition selon l'une quelconque des revendications précédentes, **caractérisée en ce que** l'alcane linéaire en C₁₃-C₄₈ est un alcane linéaire en C₁₈-C₄₀, mieux en C₂₀-C₃₂.

21. Composition selon l'une quelconque des revendications précédentes, **caractérisée en ce que** l'huile bifonctionnelle comprend de 8 à 28 atomes de carbone, mieux de 10 à 24 carbones.

22. Composition selon l'une quelconque des revendications précédentes, **caractérisée en ce que** les fonctions amide et ester de l'huile bifonctionnelle sont dans la chaîne, par exemple l'huile bifonctionnelle est le N-lauroylsarcosinate d'isopropyle.

23. Composition selon l'une quelconque des revendications précédentes, **caractérisée en ce que** les composé(s) lipophile(s) représentent de 20 à 100 %, voire de 40 % à 99 %, notamment de 60 à 95 % en poids de de la phase grasse liquide.

24. Composition selon l'une quelconque des revendications précédentes, **caractérisée en ce que** la phase grasse liquide comprend au moins une huile ester, notamment choisie parmi les esters de formule (II) suivante :
R₁-CO-O-R₂ (II)
dans laquelle :
- R₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne peut être interrompue par un ou plusieurs hétéroatomes choisis parmi N et O et/ou une ou plusieurs fonctions carbonyles, et
- R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué et dont la chaîne peut être interrompue par un ou plusieurs hétéroatomes choisis parmi N et O et/ou une ou plusieurs fonctions carbonyles.

25. Composition selon l'une quelconque des revendications précédentes, **caractérisée en ce que** la phase grasse liquide comprend au moins une huile hydrocarbonée ou un éther volatil notamment choisi(e) parmi les huiles végétales hydrocarbonées, les éthers de synthèse en C₆-C₄₀, les acides gras en C₈-C₃₂, les hydrocarbures, les fluocarbones et/ou leur mélanges.

26. Composition selon l'une quelconque des revendications précédentes, **caractérisée en ce que** la phase grasse liquide comprend une huile siliconée.

27. Composition selon l'une quelconque des revendications précédentes, **caractérisée en ce que** ladite phase grasse contient au moins de l'isododécane, de l'octyldodécanol, du N-lauroyl sarcosinate d'isopropyle, du parléam et/ou un de leur mélange et au moins une bis-urée de formule (I) dans laquelle les deux groupements R figurent respectivement un groupement : 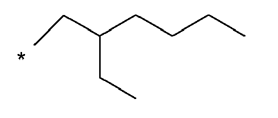 avec * tel que défini en revendication 6.

28. Composition selon l'une quelconque des revendications précédentes, **caractérisée en ce que** ladite phase grasse contient au moins de l'isododécane, de l'octyldodécanol, du parléam, du N-lauroyl sarcosinate d'isopropyle et/ou un de leurs mélanges et au moins une bis-urée de formule (I) dans laquelle les deux groupements R figurent deux groupements : 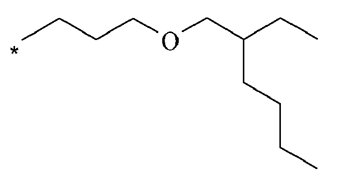 avec * tel que défini en revendication 6.

29. Composition selon l'une quelconque des revendications précédentes, **caractérisée en ce que** ledit composé de formule générale (I) dérive de la réaction entre au moins un di-isocyanate de formule : et une amine de formule : 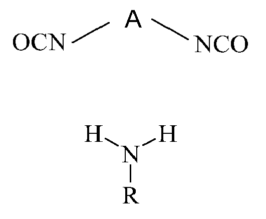 avec A et R tels que définis en revendications 1 à 14.

30. Composition selon l'une quelconque des revendications précédentes, **caractérisée en ce qu'**elle comprend en outre une matière colorante.

31. Composition selon l'une quelconque des revendications précédentes, **caractérisée en ce qu'**elle comprend des charges, des pigments, et/ou leurs mélanges.

32. Composition selon l'une quelconque des revendications précédentes, **caractérisée en ce qu'**elle contient au moins un actif cosmétique ou dermatologique.

33. Composition selon l'une quelconque des revendications précédentes, **caractérisée en ce qu'**elle contient au moins un additif choisi parmi l'eau, les oligo-éléments, les adoucissants, les hydratants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides et/ou leurs mélanges.

34. Utilisation d'un composé de formule générale (I) tel que défini selon l'une quelconque des revendications 1 à 14 et 29 pour texturer une composition cosmétique.

35. Procédé de soin et/ou de maquillage d'une matière kératinique, notamment de la peau, comprenant l'application sur la surface à traiter d'une composition selon l'une quelconque des revendications 1 à 33.
